# EUROPÄISCHE PATENTSCHRIFT

(11) **EP 2 403 418 B1**
(45) Veröffentlichungstag und Bekanntmachung des Hinweises auf die Patenterteilung: **08.05.2013**
(21) Anmeldenummer: 10711835.8
(22) Anmeldetag: 02.03.2010
(51) Int. Cl.: A61B 17/16, A61B 17/32

(54) **MEDIZINISCHES INSTRUMENT**
MEDICAL INSTRUMENT
INSTRUMENT MÉDICAL

(30) Priorität: 04.03.2009 DE 102009011205
(43) Veröffentlichungstag der Anmeldung: 11.01.2012
(73) Patentinhaber: Karl Storz GmbH & Co. KG, 78532 Tuttlingen (DE)
(72) Erfinder: SCHNEIDER, Sven, 78532 Tuttlingen (DE)
(74) Vertreter: Fugmann, Winfried
(86) Internationale Anmeldenummer: PCT/EP2010/001281
(87) Internationale Veröffentlichungsnummer: WO 2010/099930

(56) Entgegenhaltungen:
- EP-A1- 1 721 577
- WO-A1-2008/025480
- DE-A1- 10 327 655
- DE-T2- 69 024 219
- DE-U1- 9 317 535

## Beschreibung

Die Erfindung betrifft ein medizinisches Instrument mit einem hohlen Schaft, an dessen proximalem Ende eine aus mindestens zwei Griffteilen bestehende Handhabe angeordnet ist und an dessen distalem Ende über einen Kupplungsmechanismus eine Werkzeugspitze festlegbar ist, die ein aus mindestens zwei Maulteilen bestehendes, quer zur Instrumentenlängsachse abgewinkeltes Werkzeug aufweist, wobei mindestens ein Maulteil des Werkzeugs zum Öffnen und Schließen über ein verstellbar ausgebildetes Griffteil der Handhabe gegenüber dem mindestens einen anderen Maulteil des Werkzeugs verschwenkbar ist und das mindestens eine verschwenkbare Maulteil und das entsprechende, zum Verschwenken des Maulteils dienende Griffteil der Handhabe über eine axial verschiebbar in dem hohlen Schaft gelagerte Zug-/Druckstange miteinander in Wirkverbindung stehen.

Gattungsgemäße medizinische Instrumente, bei denen das verschwenkbare Maulteil ausgehend von einem verschwenkbaren Griffteil der Handhabe über ein Zug-/Druckelement betätigbar ist, finden insbesondere in der endoskopischen Chirurgie vielseitige Verwendung, beispielsweise als Stanzen, Scheren, Nadelhalter, Fassinstrumente und dergleichen.

Bei den medizinischen Instrumenten, bei denen die Maulteile des Werkzeugs seitlich zur Instrumentenlängsachse abgewinkelt ausgebildet sind, ist es notwendig, die über das Verschwenken der Handhabe initiierte Axialbewegung der Zug-/Druckstange in eine Drehbewegung umzusetzen. Hierzu ist es beispielsweise aus der DE 103 27 655 A1 bekannt, die Zug-Druckstange und das verschwenkbare Maulteil über einen Übertragungshebel miteinander zu verbinden. Diese konstruktive Umsetzung der Axialbewegung in eine Drehbewegung hat sich in der Praxis durchaus bewährt, jedoch ist diese Ausgestaltung konstruktiv sehr aufwändig.

Eine vergleichbares medizinisches Instrument gemäß dem Oberbegriff ist weiterhin aus der DE 690 24 219 T2 (Basis für den Oberbegriff des Anspruchs 1) bekannt, das sich als wenig reparaturfreundlich und schlecht sterilisierbar erweist.

Davon ausgehend liegt der Erfindung die **Aufgabe** zugrunde, ein medizinisches Instrument der eingangs genannten Art so auszugestalten, dass die Umsetzung der Axialbewegung der Zug-/Druckstange in die Drehbewegung des verschwenkbaren Maulteils bei guter Kraftübertragung einfach und zuverlässig erfolgt und dabei reparaturfreundlich bzw. sterilisierungsfreundlich aufgebaut sein soll.

Die **Lösung** dieser Aufgabenstellung ist erfindungsgemäß dadurch gekennzeichnet, dass jedes verschwenkbare Maulteil mit einer sich nach proximal erstreckenden Hülse verbunden ist, die um die Instrumentenlängsachse verdrehbar und in Axialrichtung fixiert in der Werkzeugspitze gelagert ist und, dass die Zug-/Druckstange und jede Hülse zum Verschwenken des betreffenden verschwenkbaren Maulteils über eine Zapfen-Schlitz-Steuerung in Wirkverbindung miteinander stehen.

Durch die erfindungsgemäße Lagerung des mindestens einen verschwenkbaren Maulteils an einer verdrehbar in der Werkzeugspitze gelagerten Hülse, die ihrerseits über eine Zapfen-Schlitz-Steuerung mit der Zug-/Druckstange in Wirkverbindung steht, ist es auf konstruktiv einfache Weise möglich, die Axialbewegung der Zug-/Druckstange in eine Rotationsbewegung der Hülse und somit auch des verschwenkbaren Maulteils umzuwandeln.

Um die Axialbewegung der Zug-/Druckstange möglichst spielfrei in eine Rotationsbewegung der Hülse umwandeln zu können, wird gemäß einer ersten Ausführungsform der Erfindung vorgeschlagen, dass die Zapfen-Schlitz-Steuerung aus mindestens einem ortsfest an der Zug-/Druckstange angeordneten Steuerzapfen besteht, der in eine in jeder Hülse ausgebildete wendelförmige Führungsbahn eingreift.

Gemäß der Erfindung wird vorgeschlagen, einen Kopplungsmechanismus zwischen dem hohlen Schaft und der Werkzeugspitze vorzusehen und diesen als Bajonett-Verbindung auszubilden. Dieser als Bajonett-Verbindung ausgebildeter Kopplungsmechanismus zeichnet sich dadurch aus, dass er einfach zu handhaben und robust ist. Die durch den Kopplungsmechanismus bedingte Zerlegbarkeit des medizinischen Instruments in einzelne modulare Baueinheiten erleichtert die Montage und Demontage des Instruments zu Reinigungszwecken, zu Sterilisierungszwecken, zu Reparaturzwecken und/oder zum Wechseln des Werkzeugs.

Um die Zug-/Druckstange gegen Torsion zu sichern, wird mit der Erfindung weiterhin gemäß einer bevorzugten Ausbildung der Erfindung vorgeschlagen, dass im Bereich des Kopplungsmechanismus an der Zug-/Druckstange mindestens eine Abflachung ausgebildet ist, die mit einer entsprechenden Anlagefläche an der Innenseite des den Kopplungsmechanismus aufweisenden distalen Endes des Schaftes korrespondiert. Neben der Verdrehsicherung bewirkt diese mindestens eine Abflachung an der Zug-/Druckstange zusammen mit der korrespondierenden Anlagefläche der Werkzeugspitze eine exakte Führung der Zug-/Druckstange in Axialrichtung.

Gemäß einer bevorzugten Ausführungsform sind an der Zug-/Druckstange zwei einander gegenüberliegende Steuerzapfen angeordnet, die jeweils in eine in jeder Hülse ausgebildete wendelförmige Führungsbahn eingreifen, um eine gleichmäßige Führung der Zug-/Druckstange und einen gleichmäßigen Bewegungsablauf der Bewegungsumwandlung zu gewährleisten.

Gemäß einer zweiten, alternativen erfindungsgemäßen Ausführungsform wird vorgeschlagen, dass die Zapfen-Schlitz-Steuerung aus einem ortsfest an jeder Hülse angeordneten Steuerzapfen besteht, der in jeweils eine in der Zug-/Druckstange ausgebildete wendelförmige Führungsbahn eingreift.

Um die Reibung zwischen der verdrehbar im Schaft gelagerten Hülse und der Schaftinnenseite oder zwischen zwei koaxial zueinander angeordneten Hülsen zu reduzieren wird erfindungsgemäß vorgeschlagen, dass jede Hülse auf ihrer an ihrer an einem anderen Bauteil anliegenden äußeren Mantelfläche eine gleitfähige Beschichtung aufweist. Alternativ ist es selbstverständlich auch möglich, dass die Schaftinnenseite bzw. Hülseninnenseite auf ihrer an der Hülse anliegenden inneren Mantelfläche eine gleitfähige Beschichtung aufweist. Desweiteren besteht die Möglichkeit, zwischen die relativ zueinander zu verdrehenden Bauteile eine Hülse aus einem gleitfähigen Material einzufügen. Als gleitfähiges Material ist beispielsweise Teflon ® oder dergleichen vorteilhaft.

Gemäß einer ersten praktischen Ausführungsform der Erfindung wird vorgeschlagen, dass ein Maulteil des Werkzeugs starr und das andere Maulteil des Werkzeugs verschwenkbar ausgebildet ist, wobei das starre Maulteil mit der Werkzeugspitze verbunden ist und das verschwenkbare Maulteil mit der verdrehbar in der Werkzeugspitze gelagerten Hülse verbunden ist. Bei dieser Ausführungsform bildet das beispielsweise einstückig mit der Werkzeugspitze ausgebildete starre Maulteil das distale Ende des erfindungsgemäßen medizinischen Instruments.

Gemäß einer zweiten Ausführungsform zur Ausbildung des Werkzeugs wird mit der Erfindung vorgeschlagen, dass beide Maulteile des Werkzeugs verschwenkbar ausgebildet sind, wobei jedes verschwenkbare Maulteil jeweils mit einer verdrehbar in der Werkzeugspitze gelagerten Hülse verbunden ist und die beiden Hülsen koaxial ineinander gelagert sind.

Schließlich wird mit der Erfindung vorgeschlagen, dass durch die Wahl der Steigung der wendelförmigen Führungsbahn der Öffnungswinkel der Maulteile zueinander vorgebbar ist, so dass je nach Anwendungsfall und erforderlichem Öffnungswinkel des Werkzeugs eine Hülse mit entsprechender Wendelsteigung in das erfindungsgemäße medizinische Instrument einsetzbar ist.

Die Wendelsteigung spielt darüber hinaus eine Rolle beim Kraftaufwand, der benötigt wird, um die Maulteile zu öffnen sowie bei der Geschwindigkeit, mit der die Maulteile geöffnet werden. Desweiteren lässt sich über die Anordnung der wendelförmigen Führungsbahn in der Hülse, nämlich von oben nach unten von proximal nach distal oder umgekehrt, einstellen, ob beim Öffnen oder Schließen der Handhabe das verschwenkbare Maulteil geöffnet oder geschlossen wird.

Um eine möglichst spielfreie Übertragung der Drehbewegung der mindestens einen verdrehbar in der Werkzeugspitze gelagerten Hülse auf das zugehörige verschwenkbare Maulteil zu bewirken, wird erfindungsgemäß vorgeschlagen, dass das verschwenkbare Maulteil und die Hülse, beispielsweise mittels Verkleben, verdrehfest miteinander verbunden sind.

Weitere Merkmale und Vorteile der Erfindung ergeben sich anhand der zugehörigen Zeichnungen, in denen zwei Ausführungsbeispiele eines erfindungsgemäßen medizinischen Instruments nur beispielhaft dargestellt sind, ohne die Erfindung auf diese Ausführungsbeispiele zu beschränken. In den Zeichnungen zeigt:
- Fig. 1: eine schematische Seitenansicht eines erfindungsgemäßen medizinischen Instruments;
- Fig. 2: eine perspektivische Explosionsdarstellung des Details II gemäß Fig. 1. eine erste erfindungsgemäße Ausführungsform von distal aus betrachtet darstellend;
- Fig. 3: eine teilweise montierte Darstellung des Instruments gemäß Fig. 2, von proximal aus betrachtet und
- Fig. 4: eine perspektivische Explosionsdarstellung des Details IV gemäß Fig. 1, eine zweite erfindungsgemäße Ausführungsform darstellend.

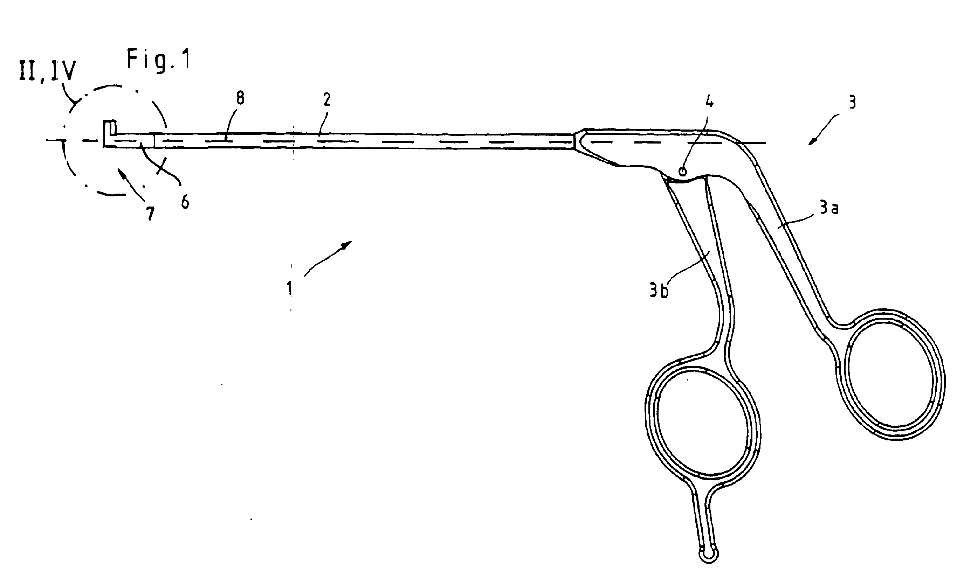

Die Abbildung Fig. 1 zeigt eine Seitenansicht eines medizinischen Instruments 1, dessen Kraftübertragungsmechanismus vielseitig verwendet werden kann, wie beispielsweise für Stanzen, Scheren, Nadelhalter, Fassinstrumente und dergleichen.

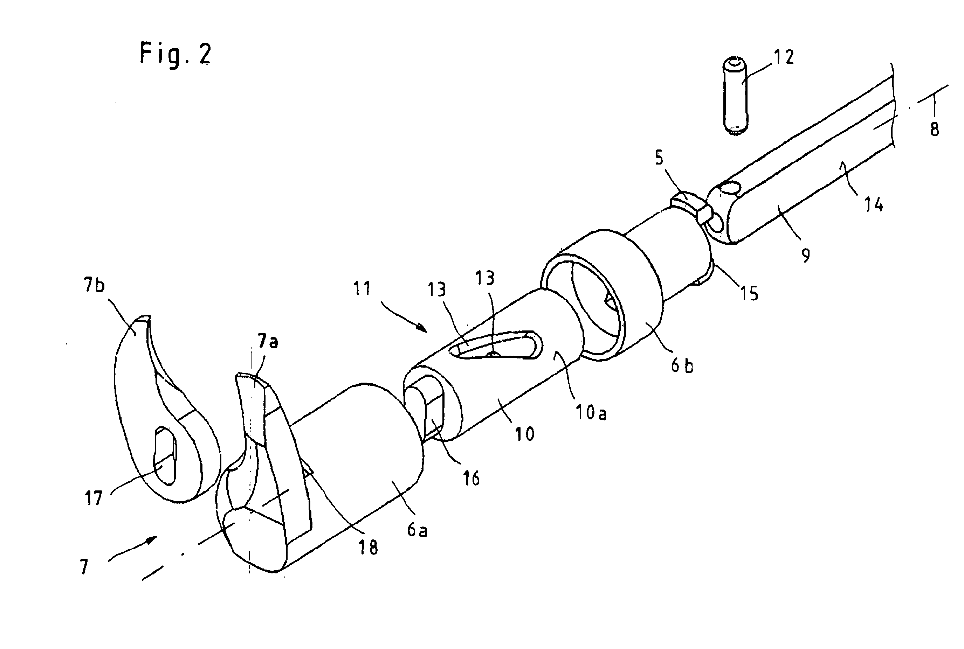

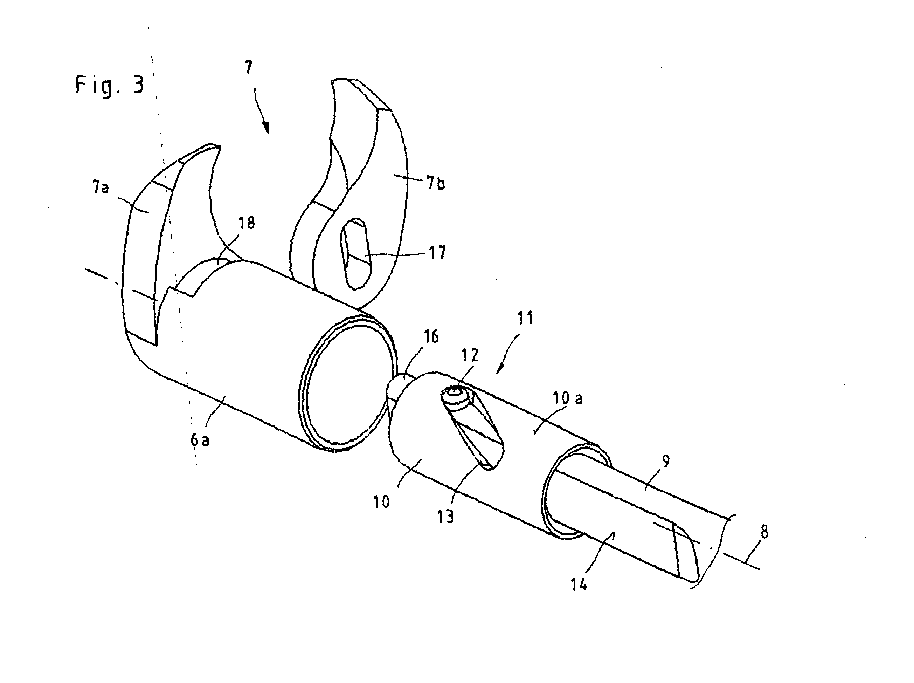

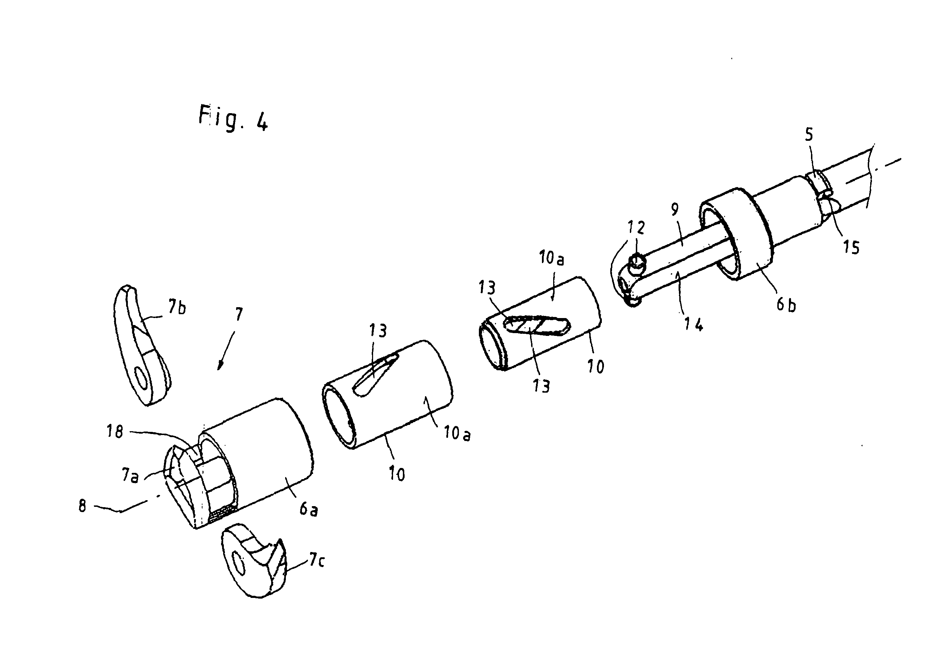

Das dargestellte medizinische Instrument 1 besteht im Wesentlichen aus einem hohlen Schaft 2, an dessen proximalem Ende eine aus einem starren Griffteil 3a und einem gegenüber dem starren Griffteil 3a um eine Schwenkachse 4 verschwenkbaren Griffteil 3b bestehende Handhabe 3 angeordnet ist. Am distalen Ende des Schaftes 2 ist über einen Kopplungsmechanismus 5 eine Werkzeugspitze 6 festlegbar, die ein Werkzeug 7 umfasst, welches beim in den Abbildungen Fig. 2 und 3 dargestellten ersten Ausführungsbeispiel aus einem starren Maulteil 7a und einem gegenüber dem starren Maulteil 7a verschwenkbaren Maulteil 7b besteht. Bei dem in Fig. 4 dargestellten zweiten Ausführungsbeispiel besteht das Werkzeug 7 aus zwei gegeneinander verschwenkbaren Maulteilen 7b und 7c.

Bei den dargestellten Ausführungsformen ist das Werkzeug 7 so ausgebildet, dass die Maulteile 7a, 7b und 7c rechtwinklig zur Instrumentenlängsachse 8 abgewinkelt sind: Andere Winkellagen der Maulteile 7a, 7b und 7c des Werkzeugs 7 zur Instrumentenlängsachse 8 zwischen 0° und 90° und auch darüber hinaus sind jedoch auch praktikabel.

Wie weiterhin aus Fig. 2 bis 4 ersichtlich, ist im hohlen Schaft 2 eine Zug-/Druckstange 9 gelagert, die distalseitig mit dem verschwenkbaren Maulteil 7b bzw. den verschwenkbaren Maulteilen 7b und 7c in Wirkverbindung steht und die proximalseitig mit dem verschwenkbaren Griffteil 3b der Handhabe 3 verbunden ist, um durch das Verschwenken des Griffteils 3b das verschwenkbare Maulteil 7b bzw. die verschwenkbaren Maulteile 7b und 7c von einer geschlossenen Stellung in eine geöffnete Stellung bzw. umgekehrt zu überführen.

Die Zerlegbarkeit des medizinischen Instruments 1 in einzelne modulare Baueinheiten, wie beispielsweise die Werkzeugspitze 6 und den Schaft 2, erleichtert die Montage und Demontage des medizinischen Instruments 1 zu Reinigungszwecken und/oder zum Wechseln des Werkzeugs 7. Bei den dargestellten Ausführungsbeispielen ist der Kopplungsmechanismus 5, über den die Werkzeugspitze 6 am Schaft 2 festlegbar ist, als Bajonett-Verbindung ausgebildet.

Um die über die Betätigung des verschwenkbaren Griffteils 3b der Handhabe 3 auf die Zug-/Druckstange 9 übertragene, ausschließlich in Axialrichtung wirkende Verschiebung der Zug-/Druckstange 9 in eine Rotationsbewegung zum Verschwenken der verschwenkbaren Maulteile 7b, 7c des Werkzeugs 7 umwandeln zu können, ist jedes verschwenkbare Maulteil 7b, 7c verdrehfest mit einer sich nach proximal erstreckenden Hülse 10 verbunden, die um die Instrumentenlängsachse 8 verdrehbar, aber in Axialrichtung fixiert in der Werkzeugspitze 6 gelagert ist, wobei die Zug-/Druckstange 9 und jede Hülse 10 zum Verschwenken der verschwenkbaren Maulteile 7b, 7c über eine Zapfen-Schlitz-Steuerung 11 in Wirkverbindung miteinander stehen.

Die Zapfen-Schlitz-Steuerung 11 besteht bei den dargestellten Ausführungsbeispielen aus zwei einander gegenüberliegend ortsfest an der Zug-/Druckstange 9 angeordneten Steuerzapfen 12, die in jeweils eine in jeder Hülse 10 ausgebildete wendelförmige Führungsbahn 13 eingreifen. Selbstverständlich ist auch die Verwendung nur eines Steuerzapfens 12 und einer wendelförmigen Führungsbahn 13 ausreichend, jedoch bieten zwei Steuerzapfen 12 eine bessere Führung der Zug-/Druckstange 9 und einen gleichmäßigen Bewegungsablauf der Bewegungsumwandlung von der translatorischen in die rotatorische Bewegung.

Alternativ zu der zuvor beschriebenen Ausgestaltung der Zapfen-Schlitz-Steuerung 10 ist es auch möglich, an der Hülse 10 einen ortsfesten Steuerzapfen 12 anzuordnen, der in jeweils eine in der Zug-/Druckstange 9 ausgebildete wendelförmige Führungsbahn 13 eingreift.

Diese wie zuvor beschrieben ausgebildeten Zapfen-Schlitz-Steuerungen 11 bewirken, dass bei der Verschiebung der Zug-/Druckstange 9 in Axialrichtung die in die wendelförmigen Führungsbahnen 13 eingreifenden Steuerzapfen 12 der Zug-/Druckstange 9 die in der Werkzeugspitze 6 gelagerten Hülsen 8 um die Instrumentenlängsachse 8 verdrehen, was seinerseits ein Verschwenken des mit den Hülsen 10 verbundenen verschwenkbaren Maulteilen 7b, 7c des Werkzeugs 7 bewirkt.

Durch die Wahl der Steigung der wendelfömigen Führungsbahnen 13 ist der Öffnungswinkel der Maulteile 7a, 7b und 7c zueinander vorgebbar, so dass je nach Anwendungsfall und erforderlichem Öffnungswinkel des Werkzeugs 7 eine Hülse 10 mit entsprechender Wendelsteigung in die Werkzeugspitze 6 des medizinischen Instruments 1 einsetzbar ist.

Die Wahl der Wendelsteigung spielt darüber hinaus eine Rolle beim Kraftaufwand, der benötigt wird, um die Maulteile 7b , 7c zu öffnen sowie bei der Geschwindigkeit, mit der die Maulteile 7b, 7c geöffnet werden. Desweiteren lässt sich über die Anordnung der wendelförmigen Führungsbahnen 13 in der Hülse 10, nämlich von oben nach unten von proximal nach distal oder umgekehrt, einstellen, ob beim Öffnen oder Schließen der Handhabe 3 die verschwenkbaren Maulteile 7b, 7c geöffnet oder geschlossen werden.

Um die Reibung zwischen der verdrehbar im Schaft 2 gelagerten Hülse 10 und der Schaftinnenseite oder zwischen zwei koaxial ineinander gelagerten Hülsen 10 (Fig. 4) zu reduzieren, ist es vorteilhaft, jede Hülse 10 auf ihrer an der Schaftinnenseite bzw. Hülseninnenseite anliegenden äußeren Mantelfläche 10a mit einer gleitfähigen Beschichtung zu versehen. Alternativ ist es auch möglich, dass die Schaftinnenseite bzw. Hülseninnenseite auf ihrer an der Hülse 10 anliegenden inneren Mantelfläche eine gleitfähige Beschichtung aufweist. Desweiteren besteht die Möglichkeit, zwischen die relativ zueinander zu verdrehenden Bauteile eine Hülse aus einem gleitfähigen Material einzufügen. Als gleitfähiges Material ist beispielsweise Teflon ® oder dergleichen vorteilhaft.

Um die Zug-/Druckstange 9 gegen Torsion zu sichern, ist im Bereich des als Bajonett-Verbindung ausgebildeten Kopplungsmechanismus 5 an der Zug-/Druckstange 9 mindestens eine Abflachung 14 ausgebildet, die mit einer entsprechenden Anlagefläche 15 an der Innenseite der den Kopplungsmechanismus aufweisenden Werkzeugspitze 6 korrespondiert. Neben der Verdrehsicherung gewährleistet diese mindestens eine Abflachung 14 an der Zug-/Druckstange 9 zusammen mit der korrespondierenden Anlagefläche 15 der Werkzeugspitze 6 eine exakte Führung der Zug-/Druckstange 9 in Axialrichtung.

Die beiden in den Abbildungen Fig. 2 und 3 sowie in Fig. 4 dargestellten medizinischen Instrumente 1 unterscheiden sich voneinander durch die Ausbildung der Werkzeuge 7, nämlich in ein Werkzeug 7 mit einem starren Maulteil 7a und einem verschwenkbaren Maulteil 7b gemäß Fig. 2 und 3 und in ein Werkzeug 7 mit zwei gegeneinander verschwenkbaren Maulteilen 7b und 7c gemäß Fig. 4.

Bei der in Fig. 2 und 3 dargestellten ersten Ausführungsform ist das starre Maulteil 7a fest mit der Werkzeugspitze 6 verbunden, während das verschwenkbare Maulteil 7b verdrehfest mit der Hülse 10 verbindbar ist, die im montierten Zustand verdrehbar in der Werkzeugspitze 6 gelagert ist. Das drehfeste Verbinden der Hülse 10 mit dem verschwenkbaren Maulteil 7b erfolgt beispielsweise durch Verkleben oder formschlüssigen Verbinden der beiden Bauteile 7b und 10, wobei bei der dargestellten Ausführungsform an der Hülse 10 distalseitig ein Zapfen 16 angeformt ist, der im montierten Zustand in eine korrespondierende Aufnahme 17 im verschwenkbaren Maulteil 7b eingreift.

Bei der in Fig. 2 und 3 dargestellten Ausführungsform besteht die Werkzeugspitze 6 aus zwei Teilen, einem die Maulteile 7a und 7b aufweisenden distalen Teil 6a sowie einem den Kopplungsmechanismus 5 aufweisenden proximalen Teil 6b. Die beiden Teile 6a und 6b der Werkzeugspitze 6 werden beispielsweise durch Verschweißen miteinander verbunden.

Zur Montage des in Fig. 2 und 3 dargestellten medizinischen Instruments 1 wird zunächst die Zug-/Druckstange 9 von proximal durch den proximalen Teil 6b der Werkzeugspitze 6 geschoben, bis die Zug-/Druckstange 9 in der Hülse 10 Aufnahme findet. In dieser Stellung wird der Steuerzapfen 12 so in die Zug-/Druckstange 9 eingesetzt, dass der Steuerzapfen 12 die Zapfen-Schlitz-Steuerung 11 bildend, in die beiden in der Hülse 10 ausgebildeten wendelförmigen Führungsbahnen 13 eingreift.

Anschließend wird das verschwenkbare Maulteil 7b über eine Aussparung 18 in die Werkzeugspitze 6 eingesetzt und die mit der Zug-/Druckstange 9 gekoppelte Hülse 10 in den distalen Teil 6a der Werkzeugspitze 6 eingeschoben, bis der Zapfen 16 der Hülse 10 in die Aufnahme 17 des verschwenkbaren Maulteils 7b eingreift.

Im nachfolgenden Montageschritt werden der proximale Teil 6b und der distale Teil 6a der Werkzeugspitze 6 miteinander verbunden und im letzten Schritt dann die Werkzeugspitze 6 und der Schaft 2 über die Bajonett-Verbindung miteinander gekoppelt.

Bei der in Fig. 4 dargestellten zweiten Ausführungsform besteht das Werkzeug 7 aus den beiden gegeneinander verschwenkbaren Maulteilen 7b und 7c, die jeweils mit einer verdrehbar in der Werkzeugspitze 6 gelagerten Hülse 10 verbunden sind, wobei die beiden Hülsen 10 im montierten Zustand koaxial ineinander und gegeneinander verdrehbar in der Werkzeugspitze 6 gelagert sind.

Das drehfeste Verbinden der Hülsen 10 mit dem verschwenkbaren Maulteilen 7b und 7c erfolgt beispielsweise durch Verkleben oder formschlüssigen Verbinden der beiden Bauteile 7b und 10 bzw. 7c und 10.

Bei der in Fig. 4 dargestellten Ausführungsform besteht die Werkzeugspitze 6 ebenfalls aus zwei Teilen, einem die Maulteile 7a und 7b aufweisenden distalen Teil 6a sowie einem den Kopplungsmechanismus 5 aufweisenden proximalen Teil 6b. Die beiden Teile 6a und 6b der Werkzeugspitze 6 werden beispielsweise durch Verschrauben miteinander verbunden.

Zur Montage des in Fig. 4 dargestellten medizinischen Instruments 1 wird zunächst die Zug-/Druckstange 9 von proximal durch den proximalen Teil 6b der Werkzeugspitze 6 geschoben, bis die Zug-/Druckstange 9 in den beiden koaxial ineinander geschobenen Hülsen 10 Aufnahme findet. In dieser Stellung wird der Steuerzapfen 12 so in die Zug-/Druckstange 9 eingesetzt, dass der Steuerzapfen 12 die Zapfen-Schlitz-Steuerung 11 bildend, jeweils in die beiden in der Hülsen 10 ausgebildeten wendelförmigen Führungsbahnen 13 eingreift.

Anschließend werden die verschwenkbaren Maulteile 7b und 7c über eine Aussparung 18 in die Werkzeugspitze 6 eingesetzt und die mit der Zug-/Druckstange 9 gekoppelten Hülsen 10 in den distalen Teil 6a der Werkzeugspitze 6 eingeschoben, bis die Hülsen 10 verdrehfest mit den verschwenkbaren Maulteilen 7b und 7c verbunden sind.

Im nachfolgenden Montageschritt werden der proximale Teil 6b und der distale Teil 6a der Werkzeugspitze 6 miteinander verbunden und im letzten Schritt dann die Werkzeugspitze 6 und der Schaft 2 über die Bajonett-Verbindung miteinander gekoppelt.

Die solchermaßen ausgebildeten medizinischen Instrumente 1 zeichnen sich dadurch aus, dass durch die beschriebenen Zapfen-Schlitz-Steuerung 11 die reine Axialbewegung der Zug-/Druckstange 9 einfach und gut dosierbar sowie im Wesentlichen spielfrei in eine Rotationsbewegung der Maulteile 7b und 7c umgesetzt wird.

### Bezugszeichenliste

| | | | |
|---|---|---|---|
| 1 | medizinisches Instrument | 10a | Mantelfläche |
| 2 | Schaft | 11 | Zapfen-Schlitz-Steuerung |
| 3 | Handhabe | 12 | Steuerzapfen |
| 3a | starres Griffteil | 13 | Führungsbahn |
| 3b | verschwenkbares Griffteil | 14 | Abflachung |
| 4 | Schwenkachse | 15 | Anlagefläche |
| 5 | Kopplungsmechanismus | 16 | Zapfen |
| 6 | Werkzeugspitze | 17 | Aufnahme |
| 6a | distaler Teil | 18 | Aussparung |
| 6b | proximaler Teil | | |
| 7 | Werkzeug | | |
| 7a | starres Maulteil | | |
| 7b | verschwenkbares Maulteil | | |
| 7c | verschwenkbares Maulteil | | |
| 8 | Instrumentenlängsachse | | |
| 9 | Zug-/Druckstange | | |
| 10 | Hülse | | |

## Patentansprüche

1. Medizinisches Instrument mit einem hohlen Schaft (2), an dessen proximalem Ende eine aus mindestens zwei Griffteilen (3a, 3b) bestehende Handhabe (3) angeordnet ist und an dessen distalem Ende eine Werkzeugspitze (6) festlegbar ist, die ein aus mindestens zwei Maulteilen (7a, 7b) bestehendes, quer zur Instrumentenlängsachse (8) abgewinkeltes Werkzeug (7) aufweist, wobei mindestens ein Maulteil (7b) des Werkzeugs (7) zum Öffnen und Schließen über ein verstellbar ausgebildetes Griffteil (3b) der Handhabe (3) gegenüber dem mindestens einen anderen Maulteil (7a) des Werkzeugs (7) verschwenkbar ist und das mindestens eine verschwenkbare Maulteil (7b) und das entsprechende, zum Verschwenken des Maulteils (7b) dienende Griffteil (3b) der Handhabe (3) über eine axial verschiebbar in dem hohlen Schaft (2) gelagerte Zug-/Druckstange (9) miteinander in Wirkverbindung stehen,
wobei jedes verschwenkbare Maulteil (7b) mit einer sich nach proximal erstreckenden Hülse (10) verbunden ist, die um die Instrumentenlängsachse (8) verdrehbar und in Axialrichtung fixiert in der Werkzeugspitze (6) gelagert ist und wobei die Zug-/Druckstange (9) und jede Hülse (10) zum Verschwenken des betreffenden verschwenkbaren Maulteils (7b) über eine Zapfen-Schlitz-Steuerung (11) in Wirkverbindung miteinander stehen,
**dadurch gekennzeichnet,**
**dass** an dem distalen Ende des hohlen Schafts (2) über einen Kopplungsmechanismus (5) eine Werkzeugspitze (6) festlegbar ist
und **dass** der Kopplungsmechanismus (5), über den die Werkzeugspitze (6) am Schaft (2) festlegbar ist, als Bajonett Verbindung ausgebildet ist.

2. Medizinisches Instrument nach Anspruch 1, **dadurch gekennzeichnet, dass** im Bereich des Kopplungsmechanismus (5) an der Zug-/Druckstange (9) mindestens eine Abflachung (14) ausgebildet ist, die mit einer entsprechenden Anlagefläche (15) an der Innenseite der den Kopplungsmechanismus (5) aufweisenden Werkzeugspitze (6) korrespondiert, um die Zug-/Druckstange (9) gegen Torsion zu sichern.

3. Medizinisches Instrument nach Anspruch 1 oder 2, **dadurch gekennzeichnet, dass** die Zapfen-Schlitz-Steuerung (11) aus mindestens einem ortsfest an der Zug-/Druckstange (9) angeordneten Steuerzapfen (12) besteht, der in eine in jeder Hülse (10) ausgebildete wendelförmige Führungsbahn (12) eingreift.

4. Medizinisches Instrument nach Anspruch 3, **dadurch gekennzeichnet, dass** an der Zug-/Druckstange (9) zwei einander gegenüberliegende Steuerzapfen (12) angeordnet sind, die jeweils in eine in jeder Hülse (10) ausgebildete wendelförmige Führungsbahn (13) eingreifen.

5. Medizinisches Instrument nach Anspruch 1 oder 2, **dadurch gekennzeichnet, dass** die Zapfen-Schlitz-Steuerung (11) aus einem ortsfest an jeder Hülse (10) angeordneten Steuerzapfen (12) besteht, der in jeweils eine in der Zug-/Druckstange (9) ausgebildete wendelförmige Führungsbahn (13) eingreift.

6. Medizinisches Instrument nach einem der Ansprüche 1 bis 5, **dadurch gekennzeichnet, dass** jede verdrehbar in der Werkzeugspitze (6) gelagerte Hülse (10) auf ihrer an einem anderen Bauteil (6 oder 10) anliegenden äußeren Mantelfläche (10a) eine gleitfähige Beschichtung aufweist.

7. Medizinisches Instrument nach einem der Ansprüche 1 bis 6, **dadurch gekennzeichnet, dass** ein Maulteil (7a) des Werkzeugs (7) starr und das andere Maulteil (7b) des Werkzeugs (7) verschwenkbar ausgebildet ist, wobei das starre Maulteil (7a) mit der Werkzeugspitze (6) verbunden ist und das verschwenkbare Maulteil (7b) mit der verdrehbar in der Werkzeugspitze (6) gelagerten Hülse (10) verbunden ist.

8. Medizinisches Instrument nach einem der Ansprüche 1 bis 6, **dadurch gekennzeichnet, dass** beide Maulteile (7b, 7c) des Werkzeugs (7) verschwenkbar ausgebildet sind, wobei jedes verschwenkbare Maulteil (7b, 7c) jeweils mit einer verdrehbar in der Werkzeugspitze (6) gelagerten Hülse (10) verbunden ist und die beiden Hülsen (10) koaxial ineinander gelagert sind.

9. Medizinisches Instrument nach einem der Ansprüche 3 bis 8, **dadurch gekennzeichnet, dass** durch die Wahl der Steigung der wendelförmigen Führungsbahn (13) der Öffnungswinkel der Maulteile (7a, 7b) zueinander vorgebbar ist.

10. Medizinisches Instrument nach einem der Ansprüche 1 bis 9, **dadurch gekennzeichnet, dass** das mindestens eine verschwenkbare Maulteil (7b) und die zugehörige Hülse (10) verdrehfest miteinander verbunden sind.

11. Medizinisches Instrument nach Anspruch 10, **dadurch gekennzeichnet, dass** das mindestens eine verschwenkbare Maulteil (7b) und die zugehörige Hülse (10) miteinander verklebt sind.

## Claims

1. Medical instrument with a hollow shaft (2), at the proximal end of which a handle (3) consisting of at least two grip parts (3a, 3b) is arranged and at the distal end of which a tool tip (6) can be fixed, the tool tip having a tool (7) which consists of at least two jaw parts (7a, 7b) and is angled transversely with respect to the longitudinal axis (8) of the instrument, wherein at least one jaw part (7b) of the tool (7) can be pivoted with respect to the at least one other jaw part (7a) of the tool (7) for opening and closing by way of an adjustably designed grip part (3b) of the handle (3), and the at least one pivotable jaw part (7b) and the corresponding grip part (3b) of the handle (3) which serves to pivot the jaw part (7b) are operatively connected to one another by way of a pull/push rod (9) mounted in an axially displaceable manner in the hollow shaft (2), wherein each pivotable jaw part (7b) is connected to a sleeve (10), which extends towards the proximal end and which is mounted in the tool tip (6) such that it can be twisted about the longitudinal axis (8) of the instrument and is fixed in the axial direction, and wherein the pull/push rod (9) and each sleeve (10) are operatively connected to one another to pivot the relevant pivotable jaw part (7b) by way of a pin-and-slot control (11),
**characterized**
**in that** a tool tip (6) can be fixed to the distal end of the hollow shaft (2) by way of a coupling mechanism (5),
and **in that** the coupling mechanism (5), by way of which the tool tip (6) can be fixed to the shaft (2), is in the form of a bayonet connection.

2. Medical instrument according to Claim 1, **characterized in that** at least one flattened portion (14) is formed on the pull/push rod (9) in the region of the coupling mechanism (5), and corresponds to a corresponding contact surface (15) on the inner side of the tool tip (6) having the coupling mechanism (5), in order to secure the pull/push rod (9) against torsion.

3. Medical instrument according to Claim 1 or 2, **characterized in that** the pin-and-slot control (11) consists of at least one control pin (12), which is arranged fixed in position on the pull/push rod (9) and engages into a helical guideway (13) formed in each sleeve (10).

4. Medical instrument according to Claim 3, **characterized in that** two control pins (12) which lie opposite one another and each engage into a helical guideway (13) formed in each sleeve (10) are arranged on the pull/push rod (9).

5. Medical instrument according to Claim 1 or 2, **characterized in that** the pin-and-slot control (11) consists of a control pin (12), which is arranged fixed in position on each sleeve (10) and engages into in each case one helical guideway (13) formed in the pull/push rod (9).

6. Medical instrument according to one of Claims 1 to 5, **characterized in that** each sleeve (10) mounted in the tool tip (6) such that it can be twisted has a coating with slip properties on the outer lateral surface (10a) thereof which bears against another component (6 or 10).

7. Medical instrument according to one of Claims 1 to 6, **characterized in that** one jaw part (7a) of the tool (7) is formed so as to be rigid and the other jaw part (7b) of the tool (7) is formed so as to be pivotable, wherein the rigid jaw part (7a) is connected to the tool tip (6) and the pivotable jaw part (7b) is connected to the sleeve (10) mounted in the tool tip (6) such that it can be twisted.

8. Medical instrument according to one of Claims 1 to 6, **characterized in that** both jaw parts (7b, 7c) of the tool (7) are formed so as to be pivotable, wherein each pivotable jaw part (7b, 7c) is connected in each case to a sleeve (10) mounted in the tool tip (6) such that it can be twisted, and the two sleeves (10) are mounted coaxially one inside the other.

9. Medical instrument according to one of Claims 3 to 8, **characterized in that** the opening angle of the jaw parts (7a, 7b) in relation to one another can be predetermined by the selection of the pitch of the helical guideway (13).

10. Medical instrument according to one of Claims 1 to 9, **characterized in that** the at least one pivotable jaw part (7b) and the associated sleeve (10) are connected to one another such that they cannot twist.

11. Medical instrument according to Claim 10, **characterized in that** the at least one pivotable jaw part (7b) and the associated sleeve (10) are adhesively bonded to one another.

## Revendications

1. Instrument médical comprenant une tige creuse (2) à l'extrémité proximale de laquelle est disposée une poignée (3) constituée d'au moins deux parties de préhension (3a, 3b) et à l'extrémité distale de laquelle peut être fixée une pointe d'outil (6) qui comprend un outil (7) constitué d'au moins deux parties de mâchoire (7a, 7b) et coudé transversalement par rapport à la direction longitudinale de l'instrument (8), au moins une partie de mâchoire (7b) de l'outil (7) pouvant être pivotée, au moyen d'une partie de préhension (3b) de la poignée (3) réalisée de manière déplaçable, par rapport à l'au moins une autre partie de mâchoire (7a) de l'outil (7) en vue de l'ouverture et de la fermeture, et l'au moins une partie de mâchoire pivotante (7b) et la partie de préhension (3b) correspondante de la poignée (3), servant au pivotement de la partie de mâchoire (7b), étant en liaison fonctionnelle l'une avec l'autre au moyen d'une barre de traction/compression (9) montée de manière axialement coulissante dans la tige creuse (2), chaque partie de mâchoire pivotante (7b) étant reliée à une douille (10) s'étendant dans la direction proximale qui est montée dans la pointe d'outil (6) de manière fixée dans la direction axiale et de manière à pouvoir tourner autour de l'axe longitudinal de l'instrument (8), et la barre de traction/compression (9) et chaque douille (10) étant en liaison fonctionnelle l'une avec l'autre au moyen d'une commande à tenon et fente (11) en vue du pivotement de la partie de mâchoire pivotante (7b) concernée,
**caractérisé**
**en ce qu'**une pointe d'outil (6) peut être fixée à l'extrémité distale de la tige creuse (2) au moyen d'un mécanisme d'accouplement (5),
et **en ce que** le mécanisme d'accouplement (5), au moyen duquel la pointe d'outil (6) peut être fixée à la tige (2), est réalisé sous forme de liaison à baïonnette.

2. Instrument médical selon la revendication 1, **caractérisé en ce qu'**au moins un aplatissement (14) est réalisé sur la barre de traction/compression (9) dans la région du mécanisme d'accouplement (5), lequel aplatissement correspond à une surface d'appui (15) correspondante sur le côté intérieur de la pointe d'outil (6) comprenant le mécanisme d'accouplement (5), afin de bloquer en torsion la barre de traction/compression (9).

3. Instrument médical selon la revendication 1 ou 2, **caractérisé en ce que** la commande à tenon et fente (11) est constituée d'au moins un tenon de commande (12) disposé de manière fixe sur la tige de traction/compression (9), lequel tenon de commande vient en prise dans une glissière de guidage hélicoïdale (13) réalisée dans chaque douille (10).

4. Instrument médical selon la revendication 3, **caractérisé en ce que** deux tenons de commande (12) opposés l'un à l'autre sont disposés sur la barre de traction/compression (9), lesquels viennent en prise respectivement dans une glissière de guidage hélicoïdale (13) réalisée dans chaque douille (10).

5. Instrument médical selon la revendication 1 ou 2, **caractérisé en ce que** la commande à tenon et fente (11) est constituée d'un tenon de commande (12) disposé de manière fixe sur chaque douille (10), lequel tenon de commande vient en prise dans une glissière de guidage hélicoïdale (13) respective réalisée dans la barre de traction/compression (9).

6. Instrument médical selon l'une quelconque des revendications 1 à 5, **caractérisé en ce que** chaque douille (10) montée à rotation dans la pointe d'outil (6) comprend un revêtement glissant sur sa surface d'enveloppe extérieure (10a) s'appliquant contre un autre composant (6 ou 10).

7. Instrument médical selon l'une quelconque des revendications 1 à 6, **caractérisé en ce qu'**une partie de mâchoire (7a) de l'outil (7) est réalisée de manière fixe et l'autre partie de mâchoire (7b) de l'outil (7) est réalisée de manière pivotante, la partie de mâchoire fixe (7a) étant reliée à la pointe d'outil (6) et la partie de mâchoire pivotante (7b) étant reliée à la douille (10) montée à rotation dans la pointe d'outil (6).

8. Instrument médical selon l'une quelconque des revendications 1 à 6, **caractérisé en ce que** les deux parties de mâchoire (7b, 7c) de l'outil (7) sont réalisées de manière pivotante, chaque partie de mâchoire pivotante (7b, 7c) étant reliée respectivement à une douille (10) montée à rotation dans la pointe d'outil (6) et les deux douilles (10) étant montées l'une dans l'autre de manière coaxiale.

9. Instrument médical selon l'une quelconque des revendications 3 à 8, **caractérisé en ce que** l'angle d'ouverture des parties de mâchoire (7a, 7b) l'une par rapport à l'autre peut être prédéfini par le choix du pas de la glissière de guidage hélicoïdale (13).

10. Instrument médical selon l'une quelconque des revendications 1 à 9, **caractérisé en ce que** l'au moins une partie de mâchoire pivotante (7b) et la douille associée (10) sont reliées l'une à l'autre de manière solidaire en rotation.

11. Instrument médical selon la revendication 10, **caractérisé en ce que** l'au moins une partie de mâchoire pivotante (7b) et la douille associée (10) sont collées l'une à l'autre.
